(19) **Europäisches Patentamt / European Patent Office / Office européen des brevets**

(11) **EP 3 760 109 A1**

(12) **EUROPEAN PATENT APPLICATION**

(43) Date of publication:
06.01.2021 Bulletin 2021/01

(51) Int Cl.:
*A61B 5/02* (2006.01)   *A61B 5/022* (2006.01)

(21) Application number: 19184049.5

(22) Date of filing: 03.07.2019

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: **Koninklijke Philips N.V.**
**5656 AG Eindhoven (NL)**

(72) Inventors:
• **BOGATU, Laura**
**5656 AE Eindhoven (NL)**

• **MUEHLSTEFF, Jens**
**5656 AE Eindhoven (NL)**
• **BRESCH, Erik**
**5656 AE Eindhoven (NL)**
• **KUENEN, Maarten Petrus Joseph**
**5656 AE Eindhoven (NL)**
• **WOERLEE, Pierre**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **CONTROL UNIT FOR DERIVING A MEASURE OF ARTERIAL COMPLIANCE**

(57)    A control unit (12) and method for deriving a measure of arterial compliance based on an acquired arterial volume variation signal and measured diastolic and systolic blood pressure measurements. An oscillometric blood pressure measurement device is used to obtain a first signal representative of arterial volume variations and to obtain blood pressure measurements. Both are measured as an applied pressure to an artery is varied by the oscillometric blood pressure measurement device. The first signal is processed to compile a dataset of values, ΔV, representative of the change in the arterial volume for set step changes, ΔP, in applied pressure, at different transmural pressure values. This set of values is numerically integrated to derive a function of arterial volume with transmural pressure. This function is differentiated to thereby derive a function of arterial compliance with transmural pressure

FIG. 7

EP 3 760 109 A1

**Description**

FIELD OF THE INVENTION

This invention relates to a control unit and method for deriving a measure of arterial compliance.

BACKGROUND OF THE INVENTION

When monitoring a patient's condition in a clinical setting, early detection of critical states of the patient is important. Common monitored parameters of a patient such as heart rate or blood pressure lack specificity in indicating particular condition or problem, and are often encumbered by a time lag between patient deterioration and change in the parameter. For example, a hypotension phase may only become apparent from a blood pressure signal after the phase has been entered, thus precluding any preventative action.

One valuable source of clinical information, which is currently under-explored, is that of arterial (or vascular) compliance. This is indicative of various parameters related to circulatory integrity. Variations in this parameter are linked to changes in blood pressure, circadian rhythms, physical activity, stress, as well as other longer-term changes caused for instance by age or lifestyle. Arterial compliance can hence reveal a large amount of information regarding health status.

Arterial compliance corresponds to the ability of arteries (specifically artery walls) to distend and contract in response to changes in arterial pressure (i.e. pressure exerted on the artery wall by flowing blood). Arterial compliance can be defined by the expression

$$C_{art}\left(P_{tm}(t)\right) = \frac{dV_{art}(t)}{dP_{art}(t)} \tag{1}$$

where $V_{art}$ is arterial volume, t is time, $P_{art}$ is arterial blood pressure and $P_{tm}$ is transmural pressure (i.e. the net pressure across the artery wall). Transmural pressure, $P_{tm}$ is defined as the arterial blood pressure, $P_{art}$, minus any applied pressure being from the outside onto the artery wall, i.e. artery internal pressure minus externally applied pressure.

Arterial blood pressure $P_{art}$ oscillates with each heart cycle, with the maximum or peak value of $P_{art}$ each cycle corresponding to systolic blood pressure (SBP) and the minimum value of $P_{art}$ each cycle corresponding to diastolic blood pressure (DBP). The SBP value is the blood pressure value at the end systole phase of the heart cycle. The DBP value is the blood pressure value at the end diastole phase of the heart cycle.

Arterial compliance is affected by many different factors, and is highly person specific. It is also a non-linear function of the transmural pressure across the artery wall ($P_{tm}$). Hence it has different values depending on the net pressure across the artery wall.

Due to its dependency on multiple physiological factors, monitoring of arterial compliance has a large number of possible applications for detecting patient deterioration.

One straightforward approach is to simply monitor changes in the arterial compliance function, and for instance present these on a display for analysis by a clinician.

Another approach is to incorporate arterial compliance information into one or more physiological models (such as that describing the Windkessel effect), for the purpose of approximating further parameters such as cardiac output or peripheral resistance (the resistance of arteries to blood flow).

In further examples, knowledge of arterial compliance can be used as a convenient (non-invasive) surrogate for blood pressure estimation.

A further example application is the use of compliance information for assessment of endothelial function (the ability of the arteries to adjust lumen size to maintain homeostasis). Usually this would be performed by means of measuring artery dimensions following an induced vascular occlusion of several minutes (flow-mediated dilation). The short term changes in blood flow produce changes in arterial lumen size, revealing information about stability of a patient (such as presence of sepsis), or progression of cardiovascular diseases. Ultrasound, photoplethysmography (PPG) or pulse arrival time (PAT) based techniques are currently employed to assess the artery dimensions.

These current techniques suffer deficiencies related to the difficulty in imaging arteries (in the case of ultrasound), and/or inconvenience for the patient, due to the fact that occlusion needs to take place for several minutes to produce a detectable change in artery size. This is uncomfortable for a patient.

Monitoring compliance may allow for more long term or ongoing assessment of endothelial response, by enabling more accurate and comfortable measurements of changes in artery size compared to current practices. In particular, duration of induced occlusion of the artery can be shortened while still achieving sufficient change in artery size for measurement of endothelial response. This allows for monitoring of endothelial response over a longer or ongoing period without causing significant discomfort for the patient.

A further example application of arterial compliance information is assessment of fluid responsiveness. Arterial compliance allows for calculating of mean systemic filling pressure for example. Mean systemic filling pressure can be derived by measurement of stop-flow forearm arterial and venous equilibrium pressures. These pressure values are input to a Guytonian model. This model allows for inferring of the mean systemic filling pressure and also the effective intravascular volume status, which contributes to the assessment of fluid responsiveness. Information about compliance permits improvement in the accuracy of the parameters used in such a model, thus leading to a better quantification of fluid responsiveness.

In a similar approach, measurement of arterial compliance could also be incorporated into assessment of limb blood flow, measured by means of venous occlusion plethysmography. This measurement is also based on employing a model which describes the physiology of the occluded limb. Accurate estimation of the model's parameters (e.g. compliance) can assist in interpreting changes in limb volume during this measurement (e.g. detection of venous thrombosis).

Hence there are many useful applications for arterial compliance.

In principle, compliance can be measured by means of high resolution imaging (e.g. ultrasound), which can reveal changes in arterial diameter as arterial pressure varies between systolic and diastolic values. However, this is highly resource intensive, as it requires highly skilled operators to perform the ultrasound scan. It is also prone to errors from potential misinterpretation of the images. It also does not allow for continuous or long-term monitoring of arterial compliance, as a single scan can naturally only be representative of compliance at one point in time.

Furthermore, if information about compliance is required over a wider range of transmural pressures, then imaging technologies would need to be calibrated with steps for applying external pressure to arteries, further complicating the measurement.

An alternative approach to ultrasound imaging is to use the (typically) already available physiological signals used in clinics for patient monitoring, e.g. electrocardiogram (ECG), PPG and/or a sphygmomanometer (blood pressure measurement cuff). These signals can give information about changes in arterial volume and pressure, enabling calculation of compliance. These approaches thus allow for more practical continuous monitoring of compliance.

A sphygmomanometer is a form of blood pressure monitoring device. It comprises an inflatable cuff with an internal inflation chamber. The cuff is wrapped around a patient's arm and by inflation of the cuff, pressure is applied to the brachial artery beneath the cuff. By monitoring pressures exerted on the cuff by the artery beneath it as the applied pressure is varied, an accurate measure of arterial blood pressure can be derived.

One form of blood pressure monitoring device is an oscillometric blood pressure measurement device. This is based on detecting oscillations (i.e. oscillometric pressure signals) in the artery wall as an applied pressure is varied. These manifest in oscillations in transmural pressure across the artery wall. The amplitudes of these oscillatory signals can be used to derive an indication of systolic and diastolic blood pressure.

It also known to derive measures of arterial volume variation using blood pressure signals obtained from oscillometric blood pressure measurement devices. Prior knowledge for instance of the elasticity of the skin contacting part of the measurement device (e.g. the inflatable cuff, where this is used) allows detected pressure changes to be processed to derive the corresponding spatial displacement, and thus the corresponding arterial volume changes.

Hence, known approaches exist for deriving arterial volume variations, and diastolic and systolic arterial pressure values, at different applied pressures (i.e. different transmural pressures), using just a single oscillometric blood pressure measurement device.

However, the discrete diastolic and systolic pressure values for each heart pulse are not sufficient to derive an accurate measure of arterial compliance over that heart pulse. This is because arterial compliance is not constant over a single heart pulse (between systole and diastole), due to the non-linearity of arterial volume with transmural pressure over ranges smaller than this (approximately 40mmHg amplitude).

Hence, typically, further arterial pressure measurements are obtained using an additional external blood pressure measurement sensor of a different type, capable of acquiring blood pressure measurements at smaller intervals over a single heart pulse. This information can then be used in combination with the derived arterial volume variation signal to directly derive arterial compliance over the course of a single heart pulse.

However, this requires use of an additional blood pressure measurement sensor, which is inconvenient for both the patient and the clinician, and adds complexity and cost to the procedure.

An improved approach to measuring arterial compliance would be desirable, capable of obtaining accurate measures of arterial compliance using measurements acquired from only a single oscillometric blood pressure measurement device.

## SUMMARY OF THE INVENTION

The invention is defined by the claims.

According to examples in accordance with an aspect of the invention, there is provided a control unit for deriving a measure of arterial compliance, operably coupleable in use with an oscillometric blood pressure measurement device, the control unit adapted to:

acquire based on use of the oscillometric blood pressure measurement device a first signal indicative of a variation in arterial volume or arterial pulse volume of an artery assumed to be in contact with the measurement device, as a pressure applied to the artery by the measurement device is varied across a range of pressures;
further acquire using the oscillometric blood pressure measurement device one or more systolic and diastolic blood pressure measurement values;

based on the first signal and acquired blood pressure measurement values, compile a dataset of values, $\Delta V$, representative of the changes in arterial volume for set step changes, $\Delta P$, in applied pressure, at different transmural pressure values,
numerically integrate the dataset of $\Delta V$ values to derive a function of arterial volume with transmural pressure, and differentiating said function of arterial volume with respect to transmural pressure, to thereby derive a function of arterial compliance with transmural pressure.

Embodiments of the invention are based on a different approach to processing the signals obtained from the oscillometric blood pressure (BP) device. Rather than considering changes in arterial volume and pressure peak-to-peak over a single pulse, changes are instead considered between points in the heart cycle of neighboring pulses (from one pulse to the next). The change in arterial volume between, e.g. common points, in consecutive pulses is much smaller than the change over the course of a single pulse. As a result, compliance can be assumed to be approximately constant between neighboring pulses. This approach thus allows arterial compliance to be derived using arterial volume measurements, and diastolic and systolic BP measurements, over multiple heart pulses, obtained from just the single oscillometric measurement device.

Embodiments of the invention propose in particular to obtain using the oscillometric blood pressure measurement device a first signal indicative of a variation in arterial volume or arterial pulse volume over the range of applied pressures.

In examples, this may then be processed in successive small step intervals, until the whole signal has been processed. Each step interval corresponds to a set step in applied pressure, $\Delta P$. Across small enough intervals, these changes in the first signal are approximately equal to the change in arterial volume across the given $\Delta P$ interval.

Hence, a set of values, $\Delta V$, is built up representative of the small changes in arterial volume attributable to a small change in applied pressure.

The change in transmural pressure over these same intervals is still needed in order to obtain compliance information. This is factored in indirectly, using the measured systolic and diastolic arterial pressure values for each heart cycle. These are not incorporated directly, but are instead incorporated in determining a transmural pressure, $P_{tm}$, to which each $\Delta V$ value corresponds. As discussed above, $P_{tm}$ = applied pressure - arterial pressure.

The diastolic and systolic blood pressure measurement values may be acquired in some examples based on the first signal, e.g. based on processing of the first signal. In some examples, just one systolic and one diastolic pressure value may be obtained for the whole measurement procedure. This makes the assumption that arterial pressure does not change significantly across the duration of the measurement procedure.

Hence it may be seen that by obtaining a set of $\Delta V$ values, and a $P_{tm}$ value to which each corresponds, arterial volume changes and corresponding transmural pressure information is brought together, with the two temporally married up via the index of applied pressure (as both the pressure measurements, and the first signal, are originally defined as a function of applied pressure).

Simple numerical integration, and then subsequent differentiation, allows this array of values to be processed into a continuous function representative of arterial compliance vs transmural pressure. For example simple numerical integration may provide absolute arterial size as a function of transmural pressure, and then subsequent differentiation may allow this array of values to be processed into a continuous function representative of arterial compliance vs transmural pressure.

In deriving this, only measurements obtained using the single oscillometric BP measurement device are used, and this is achieved by considering very small intervals in pressure and volume, and considering this by means of using data from a plurality of heart cycles, rather than just one. This is the key insight upon which embodiments of this invention may be said to be based.

The proposed approach will become clearer through more detailed explanation provided in the subsequent section.

In use, the artery is assumed to be beneath the oscillometric blood pressure measurement device. For example, the oscillometric measurement device is mounted to a part of a subject's body, covering said given artery. For example, mounted to the arm, and where the artery is the Brachial artery.

The oscillometric blood pressure measurement device may comprise an inflatable cuff. The device may comprise an internal pressure sensor for measuring a pressure inside an inflation (air) chamber of the cuff.

The oscillometric blood pressure measurement device may be a sphygmomanometer for example.

The first signal indicative of variation in arterial volume of the artery is obtained using an output from the oscillometric measurement device captured as pressure applied to the artery by the measurement device is varied across a range of pressures.

There are different options regarding what is used as the first signal.

The blood pressure measurement device may output a signal indicative of (oscillations in) absolute arterial volume over said range of applied pressure. This signal may be used as the first signal in some examples.

Alternatively, the blood pressure measurement device may in some cases output a signal indicative of variation in height of an envelope of the absolute arterial volume signal, i.e. a signal representative of variation in the peak-to-peak amplitude of absolute arterial volume as a function of applied pressure or of time. This effectively corresponds to arterial pulse pressure. This signal will be referred to in this disclosure as a volume amplitude signal. This output volume amplitude signal may be used as the first signal in some examples.

The volume amplitude signal may be understood as representing variation in arterial pulse volume, i.e. the volume of each heart pulse in the artery. The signal is indicative of variation of arterial volume via this measure of pulse volume. The first signal may hence be an absolute arterial volume signal or peak-to-peak volume amplitude signal.

In further examples, the blood pressure measurement device may output a signal indicative of (oscillations in) absolute arterial volume over said range of applied pressures, and wherein the control unit extracts from this signal of a volume amplitude signal (i.e. peak-to-peak amplitude of the output absolute volume signal). In examples, this extracted peak-to-peak volume amplitude signal may be used as the first signal.

Implementation details for using each of the absolute arterial volume signal or the volume amplitude signal as the first signal will be described in greater detail in the subsequent section.

The first signal may be represented as a waveform as a function of applied pressure, or as a function of a different variable, such as time. The requirement is simply that the signal is indicative of variation in arterial volume over a period when the applied pressure is being varied.

The absolute arterial volume oscillates up and down in value with each heart cycle. As explained, peak-to-peak volume amplitude means the difference between the maximum and minimum values of the absolute arterial volume signal at each point along signal. This can be understood as the height of the envelope of the (oscillating) absolute arterial volume signal.

The peak-to-peak amplitude hence means the difference between the maximum and minimum values of absolute arterial volume in a given cycle. The signal indicative of peak-to-peak amplitude of absolute arterial volume means therefore a signal indicative of this difference between the maximum and minimum absolute arterial volume across the range of applied pressures, i.e. for each cycle across the range of applied pressure.

The transmural pressure values to which the ΔV values correspond may be determined based on the measured systolic and diastolic blood pressure values and based on the particular applied pressure value to which the respective ΔV corresponds.

In some examples, compiling the dataset of ΔV values may comprise a stepwise processing of the first signal, stepping through the first signal in successive step intervals, ΔP, of applied pressure. Each step interval ΔP is preferably of an equal amount.

In some examples, compiling the dataset of ΔV values further comprises determining a transmural pressure value corresponding to each of the successive step intervals.

According to one or more examples, transmural pressure values to which each of the ΔV values correspond may be determined based on the measured systolic and/or diastolic blood pressure values and the particular applied pressure value at which the respective ΔV value occurs. In some examples, one of systolic or diastolic arterial pressure is chosen for determining each of the transmural pressure values. In some examples, the same one of systolic and diastolic pressure values is chosen for each of the transmural pressure values.

In some examples, the ΔP interval is selected such that the ΔV values correspond to changes in arterial volume between different heart pulses represented in the first signal, e.g. consecutive heart pulses.

In some examples, the set of values ΔV are based on changes in absolute arterial volume measured at systole or diastole for said set step changes, ΔP, in applied pressure, at different transmural pressure values. In some examples, systole may be used if applied pressure is being increased by the measurement device across said range of applied pressures. In some examples, diastole may be used if pressure is being decreased by the measurement device across said range of applied pressures.

As discussed above, the first signal may be a peak-to-peak volume amplitude signal, $V_{amp}$, indicative of a peak-to-peak amplitude of arterial volume oscillations over said range of applied pressures.

The peak-to-peak volume amplitude signal may be represented by a waveform representing the variation in peak-to-peak amplitude of the absolute arterial volume as a function of the changing applied pressure, or as a function of a different variable such as time. The requirement is simply that the signal is a variation in the peak-to-peak amplitude of the absolute arterial volume over that period when the applied pressure is being varied.

As also discussed above, in some examples, the signal output from the blood pressure measurement device is representative of absolute arterial volume, and wherein the control unit is configured to extract from said signal a peak-to-peak volume amplitude signal, $V_{amp}$, indicative of a peak-to-peak amplitude of the arterial volume across said range of applied pressures, this $V_{amp}$ signal being used as the first signal.

In some examples, the set of values $\Delta V$ are based on changes in the volume amplitude function, $V_{amp}$, for said set step changes, $\Delta P$, in applied pressure, at different transmural pressure values.

In preferred examples, the oscillometric blood pressure measurement device comprises a fluid-inflatable cuff.

In some embodiments, in use, the control unit is configured to implement said variation in applied pressures across a range of pressures by controlling the inflatable cuff to gradually inflate or deflate through a range of internal fluid pressures of the inflatable cuff.

In some embodiments, the applied pressure to the artery is taken to be equal to a baseline value of an internal fluid pressure of the inflatable cuff. This simplifies the procedure and does not reduce accuracy of derived measures of compliance by any significant degree.

In some embodiments, the first signal is obtained based on a combination of: measured oscillations in an internal fluid pressure of the fluid-inflatable cuff at each applied pressure value, and a pre-determined internal fluid pressure vs internal fluid volume relationship.

The pre-determined internal fluid pressure vs internal fluid volume relationship may in some examples utilize a pre-determined compliance or elasticity of the fluid-inflatable cuff.

Examples in accordance with a further aspect of the invention provide a control unit in accordance with any example or embodiment outlined above or described below, or in accordance with any claim of this application, and an oscillometric blood pressure measurement device operatively coupled to the control unit.

In some embodiments, the system may further comprise a patient monitor unit, wherein the patient monitor unit comprises the control unit.

In some embodiments, the system may be a patient monitoring system. It may for example comprise one or more input ports for receiving data from one or more further auxiliary units, e.g. medical sensing devices.

Examples in accordance with a further aspect of the invention provide a method of deriving a measure of arterial compliance, comprising:

obtaining based on use of an oscillometric blood pressure measurement device a first signal indicative of a variation in arterial volume or arterial pulse volume of an artery assumed to be in contact with the measurement device, as a pressure applied to the artery by the measurement device is varied across a range of pressures;

further obtaining using the oscillometric blood pressure measurement device one or more systolic and diastolic blood pressure measurement values;

based on the first signal, and the acquired blood pressure measurement values, compiling a dataset of values, $\Delta V$, representative of the change in arterial volume for set step changes, $\Delta P$, in applied pressure, at different transmural pressure values,

numerically integrating the dataset of $\Delta V$ values to derive a function of arterial volume with transmural pressure, and differentiating said function of arterial volume with respect to transmural pressure, to thereby derive a function of arterial compliance with transmural pressure.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates an example inflatable cuff of an example oscillometric blood pressure measurement device;
Fig. 2 illustrates an example oscillometric blood pressure measurement device in use;
Fig. 3 illustrates a cross-section through an example artery and applied inflatable blood pressure measurement cuff;
Fig. 4 shows pressure oscillations in an internal inflation chamber of an inflatable cuff as a function of time in an example implementation of a control unit according to one or more embodiments;
Fig. 5 shows derived arterial volume oscillations as a function of applied pressure in an example implementation according to one or more embodiments;
Fig. 6 shows an extracted signal indicative of variation in peak-to-peak amplitude of the arterial volume signal of Fig. 5;

Fig. 7 illustrates a typical relationship between arterial volume and transmural pressure;
Fig. 8 illustrates differing ranges of transmural pressure covered by successive heart pulses;
Fig. 9 shows an algorithm for deriving an array of values corresponding to changes in arterial volume for set intervals in applied pressure at different transmural pressures;
Fig. 10 shows an arterial volume vs transmural pressure function derived by integrating the array of values provided as an output of the algorithm of Fig. 9;
Fig. 11 shows an example arterial compliance vs transmural pressure function derived by differentiating the function of Fig. 10; and
Fig. 12 shows example comparisons of arterial volume vs transmural pressure functions derived using an embodiment of the present invention, and using a parametric model based method.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an improved means for deriving a measure of arterial compliance. The proposed approach enables deriving arterial compliance using measurements obtained using a single oscillometric blood pressure measurement device only. A processing procedure is applied to obtained measurements to derive arterial compliance.

Deriving arterial compliance allows for faster detection of patient deterioration. Furthermore, embodiments allow for detecting arterial compliance based on measurements obtained non-invasively from the same site of the body (e.g. brachial artery). Compared to similar measurements acquired from a more peripheral site, such as. a finger site, this method processes allows for deriving hemodynamic parameters more representative of the whole cardiac or hemodynamic system. Larger arteries for example are less affected by local peripheral factors such as cold temperatures at extremities.

Embodiments according to the present invention permit, inter alia improvement in the following applications:

- Earlier detection of patient deterioration: changes in arterial compliance often precede large changes in blood pressure and hemodynamic instability.
- Diagnosis and treatment of cardiovascular diseases: measurement of arterial compliance can play a role in assessment of cardiovascular disease progression and in evaluation of patient responsiveness to treatment.
- Improved accuracy of calibration of blood pressure surrogate measures such as pulse arrival time and pulse morphology features.

Accurate and, for instance, long-term or ongoing or continuous measurement of arterial compliance also may at least partially assist in the development of personalized medical procedures, since arterial compliance is one useful factor in better identifying different patient groups, e.g. groups distinguished in terms of medication responses.

An example control unit in accordance with one or more embodiments will now be described in detail. The control unit is for deriving a measure of arterial compliance, operably coupleable in use with an oscillometric blood pressure measurement device.

The control unit 12 is adapted to perform a series of steps which when performed result in deriving of an output measure indicative of arterial compliance.

The series of steps performed by the control unit 12 will now be outlined in summary, followed by a more detailed explanation of different implementation options for each step.

The control unit 12 is adapted to obtain using the oscillometric blood pressure measurement device 14 a first signal indicative of a variation in arterial volume or arterial pulse volume of an artery assumed to be in contact with the measurement device, as a pressure applied to the artery by the measurement device is varied across a range of pressures.

The control unit 12 may control the oscillometric measurement device 14 to vary the applied pressure through said range of pressure. Alternatively the oscillometric blood pressure measurement device 14 may incorporate a local controller for controlling the applied pressure variation, or a further auxiliary controller may be provided for controlling the pressure for instance.

There are different options regarding what signal is used as said first signal. The first signal may be a signal representative of variation in (i.e. oscillations in) absolute arterial volume as a function of applied pressure or of time.

Alternatively, the first signal may be representative of variation in peak-to-peak amplitude of absolute arterial volume oscillations over the range of applied pressures. In such a case the first signal effectively represents a pulse volume signal.

The blood pressure measurement device may directly output a signal representative of absolute arterial pressure oscillations over the range of applied pressure. This may be used as the first signal, or may be further processed to obtain a further signal representative of peak-to-peak amplitude of this signal, and this further signal used as the first signal. Alternatively, the pressure measurement device may directly output a signal representative of peak-to-peak amplitude of absolute arterial oscillations over the range of applied pressures, and this used as the first signal.

The measurement device may include a fluid-inflatable cuff, or the cuff may be external to the device, and wherein the controller is operatively coupleable to such a cuff. The cuff in use may be gradually inflated so as to increase a pressure applied to the artery through a range of ever-increasing pressures. Alternatively, the cuff may in use be inflated to fully occlude the artery in question before the applied pressure is gradually reduced through a range of decreasing applied pressures.

The cuff may be adapted to sense arterial volume oscillations of the artery as applied pressure is varied. These volume oscillations naturally depend on arterial compliance (distension of the artery wall in response to transmural pressure changes).

The measurement device may measure changes, e.g. oscillations, in internal fluid pressure within an inflation chamber in the cuff. For a given (fixed) cuff inflation, such oscillations can be taken to be representative of variations in pressure applied to the cuff by the underlying artery as it pulses with blood.

If the compliance (elasticity) of the cuff is known, this can be converted into a signal indicative of absolute arterial volume variations of blood within the artery at each of the range of different applied pressures. It may additionally or alternatively be converted into a signal representative of variation in peak-to-peak amplitude of absolute arterial volume.

Other pressure sensing means could be used in further examples, for instance simply a contact pressure sensor or transducer arranged to sense pressures applied to a surface of the cuff.

A first signal is obtained based on an output of the oscillometric blood pressure measurement device, the first signal indicative of a variation in arterial volume of an artery assumed to be in contact with the measurement device, as a pressure applied to the artery by the measurement device is varied across a range of pressures. The first signal may be representative of absolute arterial volume oscillations, or of variations in peak-to-peak amplitude of the absolute volume oscillations (i.e. pulse volume).

In either case, based on the first signal and further acquired blood pressure measurement values, a dataset of values is compiled, $\Delta V$, representative of the changes in arterial volume for set step changes, $\Delta P$, in applied pressure, at different transmural pressure values.

The control unit then numerically integrates the dataset of $\Delta V$ values to derive a function of absolute arterial volume with transmural pressure. The control unit then differentiates said function of arterial volume with respect to transmural pressure, to thereby derive a function of arterial compliance with transmural pressure.

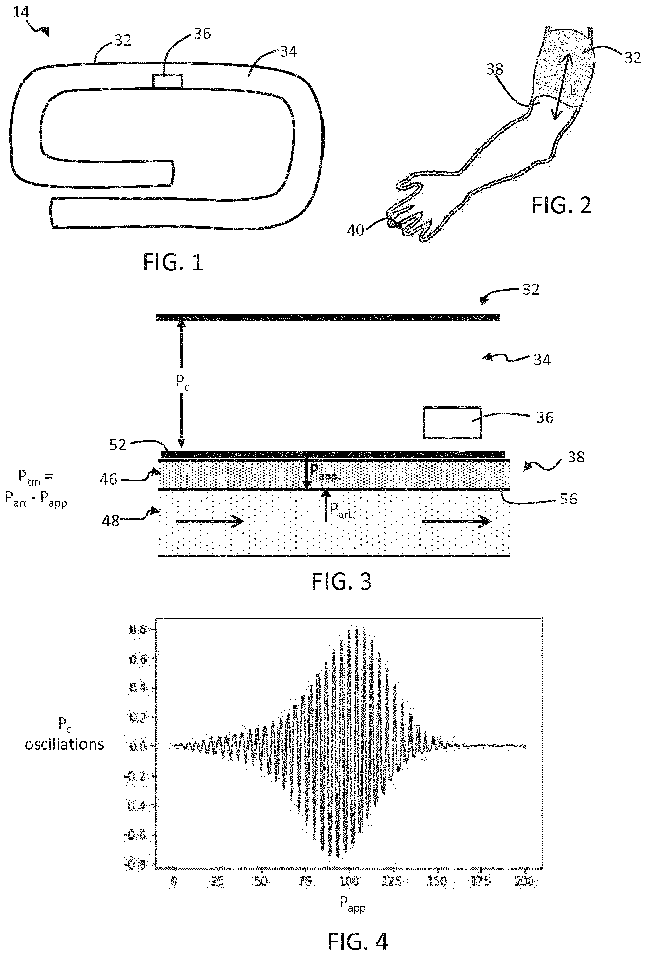

By way of brief explanation, Fig. 1 shows a cross-sectional view through an example oscillometric blood pressure measurement device 14 which may in examples be used with the control unit in use.

The oscillometric measurement device 14 in this example is in the form of a sphygmomanometer.

The example oscillometric measurement device 14 comprises an inflatable cuff 32 with an internal air chamber 34 with a volume which can be varied to change an inflation level. The air chamber can in other examples be any fluid chamber. The internal air chamber accommodates an air pressure sensor 36 which senses an internal cuff pressure.

In use, the inflatable cuff is wrapped around a part of a subject's body, most typically the upper arm, such that the cuff overlies, and is arranged for applying pressure to, an artery included in said part of the body.

This is illustrated in Fig. 2 which shows the cuff 32 applied to the upper arm 38. In this case the cuff overlies the Brachial artery.

The control unit 12 may be adapted in use to control, e.g. issue control commands to, the oscillometric blood pressure measurement device, to acquire said signal indicative of variation in arterial volume of an artery. Alternatively the oscillometric blood pressure measurement device 14 may be independently controlled, and the control unit is configured to receive signals output from the measurement device.

Although an air pressure sensor 36 is shown in Fig. 1 for sensing the pressure oscillations, in other examples other means can be used to sense the changes in the pressure applied to the cuff by the artery 48. These include for instance a contact pressure sensor mechanically communicating with the wall 52 of the cuff for directly sensing the changing pressure applied to the wall by the artery. In other examples, a transducer such as a microphone may be used to sense vibrations in the wall caused by the pressures exerted.

The cuff may be inflated until the artery is completely occluded. The pressure applied by the cuff is then gradually decreased through a range of decreasing applied pressures, and the internal cuff pressure simultaneously (and preferably continuously) measured. This information may be used to derive a signal indicative of variation in arterial volume which is output by the measurement device. Alternatively, the cuff may instead be gradually inflated, so that pressure applied by the cuff to the artery is gradually increased through a range of increasing applied pressures.

One example implementation of the steps outlined above will now be described in more detail. This is presented by way of example only, for the purpose of demonstrating the concepts of the invention, and is not limiting of the inventive concept in general.

Fig. 3 shows a cross-sectional illustration of application of an example oscillometric blood pressure measurement device (in the form of an inflatable cuff 32) to an example section of an upper arm 38 of a subject. In particular, only an upper-most layer part of the subject's arm is shown, comprising the brachial artery 48 (by way of example) and an overlying layer of arm tissue 46.

An outer wall 52 of the cuff is shown applied to the upper surface of the arm. An internal air chamber 34 of the cuff is illustrated, and a pressure sensor 36 is accommodated within the cuff air chamber adapted to measure a cuff air pressure $P_c$ inside the air chamber. This pressure inside the chamber will be referred to for brevity as 'cuff pressure', $P_c$.

The cuff pressure $P_c$ is the pressure exerted internally within the cuff air chamber 34 on internal surfaces of the walls 52 of the cuff chamber 34.

In operation, the inflatable cuff 32 is fitted to the subject (for instance to the upper arm), by wrapping snugly around the arm.

The cuff is then gradually inflated by gradually increasing a volume inside the cuff inflation chamber 34, thereby gradually increasing the internal cuff pressure $P_c$.

As the cuff is inflated, it exerts an increasing applied pressure, $P_{app}$, to the artery 48, via the upper tissue layer 46. This pressure applied by the cuff to the artery 48 wall will be referred to for brevity as the applied pressure, $P_{app}$.

The applied pressure, $P_{app}$, is the pressure applied by the cuff to the boundary wall 56 of the artery 48.

The applied pressure, $P_{app}$, is often assumed to be approximately equal to the internal cuff pressure $P_c$, (or for instance a baseline value of the internal cuff pressure) i.e. the pressure inside the inflation chamber of the cuff. Internal cuff pressure (or a baseline value of internal cuff pressure) may thus be used as a measure of applied pressure in some examples.

Inside the artery 48, blood flows along the lumen, pumped from the heart. The internally flowing blood exerts a pressure outward onto the interior surfaces of the boundary walls 56 of the artery 48. This pressure will be referred to as the arterial pressure, $P_{art}$, and is indicated by an arrow in Fig. 3.

The arterial pressure, $P_{art}$, oscillates up and down in value each heart cycle. The pressure reaches a maximal level at end systole, and falls to a minimum value at end diastole. These maximum and minimum arterial pressure values, associated with the different phases of the heart, are known as systolic arterial blood pressure $P_{sys}$ and diastolic arterial blood pressure $P_{dia}$ respectively.

The transmural pressure $P_{tm}$ across the artery wall 56 is equal to the difference between the arterial pressure $P_{art}$ and the applied pressure $P_{app}$. This can be expressed as

$$P_{tm} = P_{art} - P_{app}$$

In operation, the cuff is gradually inflated by gradually increasing a volume inside the cuff inflation chamber 34, thereby gradually increasing a baseline of the internal cuff pressure $P_c$. This results in a gradually increasing applied pressure $P_{app}$ to the artery 48. Alternatively the cuff may be inflated to occlude the artery and then gradually deflated through a range of decreasing pressures, thereby gradually decreasing a baseline of the internal cuff pressure.

At a minimum applied pressure, the artery 48 is fully open for example. At a maximum applied pressure the artery is fully occluded for example.

As the applied pressure, $P_{app}$, is increased, the artery becomes increasingly compressed by the cuff, thereby reducing a local internal diameter of the artery 48 lumen. The artery is thus gradually transitioned from completely open to completely occluded (or vice versa if pressure is decreased from a maximum level instead).

The local arterial pressure at the part of the artery wall 56 being compressed oscillates with certain amplitudes depending upon the transmural pressure at a given time, and upon the systolic and diastolic arterial blood pressure values in the absence of any occlusion.

The oscillations in arterial pressure $P_{art}$ can be sensed at the inflatable cuff 32 of the blood pressure measurement device since they exert an oscillating pressure to the wall 52 of the cuff inflation chamber 34. This oscillating pressure leads to small local oscillations in internal cuff pressure $P_c$. These oscillations are superposed on the background or baseline internal cuff pressure, and are sensed by the cuff pressure sensor 36.

By sensing the amplitude of the cuff pressure $P_c$ oscillations, changes in absolute arterial volume as a function of time can be determined. This will now be further explained with reference to Fig. 4.

Fig. 4 shows a waveform representing example de-trended oscillations in cuff pressure $P_c$ (y-axis; mmHg) as would be sensed inside the inflation chamber 34 of the inflatable cuff 32 as a result of the locally oscillating arterial pressure $P_{art}$. The oscillations are de-trended, meaning extracted from the overall varying global (or baseline) pressure inside the cuff chamber, varying due the cuffs gradual inflation. Hence the cuff pressure oscillation value of 0 in the illustrated waveform is calibrated as being equal to the baseline absolute cuff pressure regardless of inflation level.

The oscillations in cuff pressure $\Delta P_c$ (y-axis) are shown as a function of cuff applied pressure $P_{app}$ (x-axis; mmHg). The applied pressure may be taken as being equal to the baseline internal cuff pressure, as explained above. Hence, the applied pressure may be given by the baseline internal fluid pressure of the cuff.

If the compliance, e.g. elasticity, of the cuff 32 is known in advance, the sensed cuff pressure $P_c$ oscillations (shown in Fig. 4) can also be converted into corresponding absolute arterial volume, $V_{art}$, oscillations. The local arterial blood pressure oscillations as a function of time are merely a reflection of locally oscillating blood volumes in the compressed section of the artery. These arterial pressure oscillations are directly reflected as oscillations in internal cuff pressure $P_c$ (as explained above, via pressing of the artery on the cuff). Hence, the sensed internal cuff pressure oscillations, $P_c$, can be readily processed into absolute arterial volume oscillations if the cuff elasticity is known.

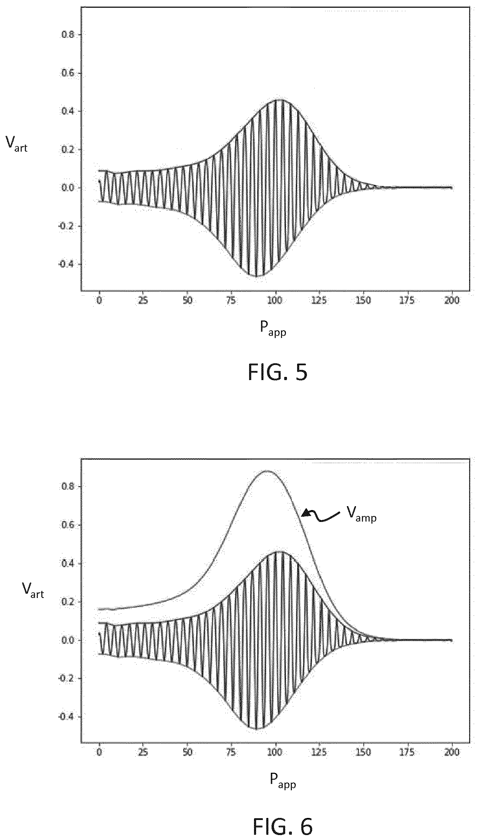

The resulting signal indicative of variation in absolute arterial volume $V_{art}$ (y-axis; units: ml) for this example is shown in Fig. 5. This signal is shown as a function of applied pressure $P_{app}$. In other examples however, it may be represented by a signal as a function of a different variable such as time. In either case, the signal represents arterial volume variations or oscillations across that period when the applied pressure is being varied by the blood pressure measurement cuff 32.

As can be seen, the signal embodies the local oscillations in volume between systole and diastole heart phases. The signal embodies the same de-trending as in the cuff pressure oscillations signal of Fig. 4. Hence, the signal shows the oscillation in absolute arterial volume around baseline arterial volume. The baseline can be expected in reality to decline gradually as cuff applied pressure $P_{app}$ is gradually increased and the local artery 48 diameter is decreased (or to increase gradually as applied pressure is decreased in examples in the cuff is gradually deflated).

As discussed above, this signal indicative of absolute arterial volume oscillations over the range of applied pressures may in some examples be used as the first signal for subsequent processing for compiling the dataset of values $\Delta V$, and the measure of arterial compliance.

For example, the set of values $\Delta V$ extracted from the first signal may be based on changes in absolute arterial volume between common fixed points in different (e.g. consecutive) oscillations of the absolute arterial volume signal. For example, the $\Delta V$ values may correspond to changes in absolute arterial volume between one of either end systole or end-diastole points (i.e. maximum or minimum points) of different volume oscillations of the signal for said set step changes, $\Delta P$, in applied pressure, at different transmural pressure values.

As also discussed above, in alternative examples, a further signal, indicative of variation in peak-to-peak amplitude of the absolute arterial volume oscillations may be instead used as the first signal for subsequent processing to derive the $\Delta V$ values.

In particular, processing whole volume waveforms may leave resulting calculations more vulnerable to noise in the signal. Accuracy or robustness of resulting compliance measurements can be increased by considering only the upper and lower peaks of the arterial volume oscillations, i.e. the systolic and diastolic volumes, or the upper and lower envelopes of the signal shown in Fig. 5.

Accordingly, there may be extracted from the signal indicative of variation in absolute arterial volume (in this example, the signal of Fig. 5) a signal indicative of peak-to-peak amplitude of arterial volume across the range of applied pressures. This corresponds to the 'height' of the envelope of the signal of Fig. 5, i.e. the upper envelope minus the lower envelope, as a function of applied pressure $P_{app}$ (in this example). Envelope here is used in its normal meaning when applied to a signal, and corresponds for instance to a pair of smooth curves outlining the extremes of the volume signal. The volume amplitude signal hence effectively corresponds to the variation in the total change in arterial volume between systole and diastole phases, across said range of applied pressures. This effectively corresponds to arterial pulse volume (the local arterial volume of each blood pulse). The peak-to-peak volume amplitude signal represents the variation in peak-to-peak amplitude of arterial volume across the range applied pressures $P_{app}$.

Extraction of a peak-to-peak amplitude signal may be performed by the control unit based on an absolute arterial volume signal received from the blood pressure measurement device. Alternatively, the blood pressure measurement device may itself derive a peak-to-peak amplitude signal and output this directly for receipt by the control unit.

A resulting extracted peak-to-peak amplitude signal ($V_{amp}$) for the example arterial volume signal of Fig. 5 is shown in Fig. 6. This shows the peak-to-peak arterial volume amplitude signal, $V_{amp}$ (y-axis; units: ml) as a function of applied pressure, $P_{app}$. However, in other examples, the signal may be represented as a function of a different variable, for example as a function of time. The requirement is simply that the signal is a variation in the peak-to-peak amplitude of the arterial volume over that period when the applied pressure is being varied by the blood pressure measurement cuff 32.

The control unit 12 is further configured to acquire, using the oscillometric blood pressure measurement device, one or more systolic and diastolic blood pressure measurement values corresponding to the same range of applied pressures $P_{app}$ for which the volume measurements are obtained. These can be acquired at the same time as the internal cuff pressure oscillations (Fig. 4) are sensed, or may be based on post-processing of the first signal for example. These may be measured at each applied pressure, but more commonly, a single systolic and a single diastolic blood pressure value is derived from the whole measurement procedure. The subsequent steps of the present procedure (described below) then assume that arterial pressure does not change to any significant extent over the duration of the measurement procedure (and hence over the span of the obtained first signal discussed above).

Acquiring diastolic and systolic blood pressure values using an oscillometric blood pressure measurement device is a well-known procedure, applied in all oscillometric blood pressure measurement devices. This disclosure will not therefore outline the procedure in any detail, and the skilled person will be aware of means for deriving these measurements. It may be based for example on processing of the first signal, for example processing of a peak-to-peak volume amplitude signal of the kind discussed above.

The arterial volume information of Fig. 5 or Fig. 6 may be used together with the systolic and diastolic arterial pressure measurements mentioned above in order to obtain a measure of arterial compliance.

However, as discussed, the only information about arterial pressure which is obtained from oscillometry are the single systolic ($P_{sys}$) and diastolic ($P_{dia}$) blood pressure values.

Given that the total arterial volume change at each applied cuff pressure $P_{app}$ is caused by a change in arterial pressure of $P_{sys}$- $P_{dia}$ (pulse pressure), one might, naively, consider obtaining compliance (as a function of applied pressure $P_{app}$) by simply dividing the peak-to-peak volume amplitude function $V_{amp}$ (Fig. 6) by the pulse pressure.

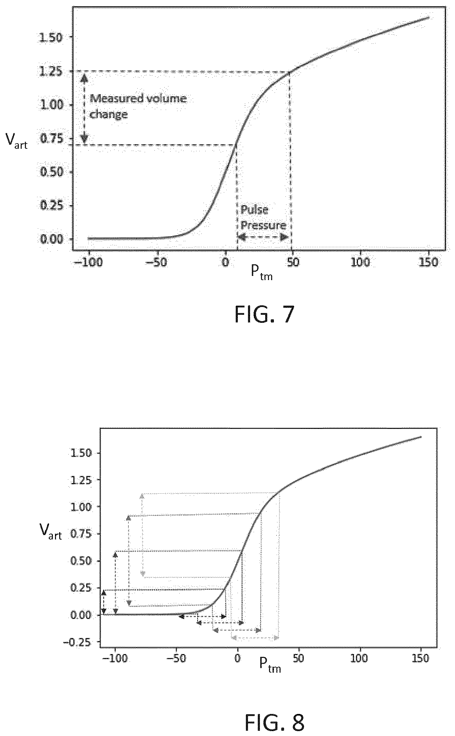

However, arterial compliance is not constant over a single pulse (approximately 40mmHg amplitude), due to the non-linearity of arterial volume with transmural pressure over ranges smaller than this. See for example Fig. 7 which shows the typical relationship between measured arterial volume (y-axis) as a function of transmural pressure (x-axis).

Hence, this naive division approach does not work in practice. A more subtle approach is required, which will now be explained in more detail.

Measurable arterial volume variations are the result of successive blood pressure pulses generating volume pulses in the artery across the duration of the cuff inflation. With every heartbeat, the transmural pressure across the artery wall thus varies between $P_{tm} = P_{sys} - P_{cuff}$ (maximum value) and $P_{tm} = P_{dia} - P_{cuff}$ (minimum value), where for the present explanation, $P_{cuff}$ (cuff internal pressure) is taken as being equal to, and thus representative of, applied pressure $P_{app}$. This results in arterial volume pulsations of amplitudes which depend on the relationship between arterial volume and transmural pressure (Fig. 7).

Given that the change in $P_{app}$ is relatively slow compared to (the fast-cycling) changes in arterial pressures, neighboring heart pulses will typically cover similar ranges of transmural pressures over the course of the given pulse (i.e. the peak-to-peak amplitudes of the pressure pulses will be similar). Thus, the changes in the first signal (absolute arterial volume or arterial pulse volume) from one pulse to another can be attributed to the small differences in applied pressures covered by consecutive beats.

This insight can be applied to derive a relationship between arterial volume (or arterial pulse volume) and transmural pressure.

In particular, the obtained first signal can be processed, starting e.g. from large applied pressure (where arterial volume is 0 ml), and small changes in volume between different applied pressures $P_{app}$ attributed to corresponding transmural pressure changes.

As illustrated by Fig. 8, every neighboring pair of heart pulses will cover a slightly different range of transmural pressures in comparison to the previous pair of heart pulses. This information can be used when analyzing the change in volume from one pulse to another. In this way, the change in volume can be more precisely attributed to a corresponding change in transmural pressure.

In more detail, the control unit is configured to, based on the first signal and the set of blood pressure measurement values at different applied pressures, compile a dataset of values, $\Delta V$, representative of the changes in arterial volume or changes in arterial pulse volume for set step changes, $\Delta P$, in applied pressure, at different transmural pressure values. This can be performed whether the first signal is representative of absolute arterial volume or is representative of peak-to-peak amplitude of absolute arterial volume oscillations (pulse volume).

The value of $\Delta P$ may be chosen such that each step $\Delta P$ will be large enough to move to a different oscillation (i.e. different heart pulse) in the first signal. In this way, each $\Delta V$ value corresponds to a change in arterial volume between different heart pulses represented in the first signal. However in other cases, and more generally, $\Delta P$ can be anywhere up to infinitely small (i.e. an infinitesimally small interval dV). For example, the first signal can be interpolated between beats, and thus it is not essential that $\Delta P$ is large enough to move to a new beat (new pulse) each time.

The procedure comprises stepwise processing of the first signal, stepping through the first signal in successive step intervals, $\Delta P$, of applied pressure. For each step interval $\Delta P$, a corresponding change $\Delta V$ in the first signal is determined, and the transmural pressure value to which the step corresponds is determined. If absolute arterial volume is used for the first signal, the change $\Delta V$ may be a change in absolute arterial volume between corresponding fixed points in the heart cycles between which the step $\Delta P$ moves. For example, the $\Delta V$ values may be based on changes in absolute arterial volume measured at systole or diastole for said set step changes, $\Delta P$, in applied pressure, at different transmural pressure values.

Transmural pressure values to which each of the $\Delta V$ values correspond may be determined based on the measured systolic and/or diastolic blood pressure values at the particular applied pressure value to which the respective $\Delta V$ value corresponds and based on the applied pressure value to which the respective $\Delta V$ value corresponds.

By way of example, the transmural pressure value for each step interval $\Delta P$, extending between applied pressure values of $P_{app\_initial}$ and $P_{app\_initial} + \Delta P$, may be taken as equal to $P_{tm} = P_{sys\_initial} - P_{app\_initial}$, where $P_{sys\_initial}$ is the systolic blood pressure for the blood pulse measured in the first signal at $P_{app\_initial}$.

A processing procedure is hence applied which is based on processing the first signal in terms of a chosen step pressure $\Delta P$. The change in volume ($\Delta V$) caused by the change in pressure $\Delta P$ is computed for absolute transmural pressures.

The output of this procedure is an array $\Delta V(P_{tm})$ which represents arterial volume changes caused by a set pressure change of value $\Delta P$ at different transmural pressures.

A plot of arterial volume as a function of transmural pressure may be obtained by then numerically integrating the values of the obtained array $\Delta V(P_{tm})$ with respect to transmural pressure. This may then be differentiated with respect to transmural pressure to obtain a signal indicative of arterial compliance as a function of transmural pressure.

A first example algorithm for deriving the dataset of values $\Delta V$, based on this approach will now be outlined.

In this example, the first signal is taken to be a signal representative of peak-to-peak amplitude of absolute arterial volume oscillations (e.g. as shown in Fig. 6). The example algorithm receives as an input a peak-to-peak volume amplitude signal $V_{amp}$ (obtained from a cuff internal pressure recording), and corresponding systolic and diastolic arterial pressure values. The measurements are assumed to have been obtained using a blood pressure measurement cuff in which applied pressure is gradually decreased from a starting maximum pressure $P_{cuffmax}$ through a range of decreasing pressures to a minimum pressure P cuffmin.

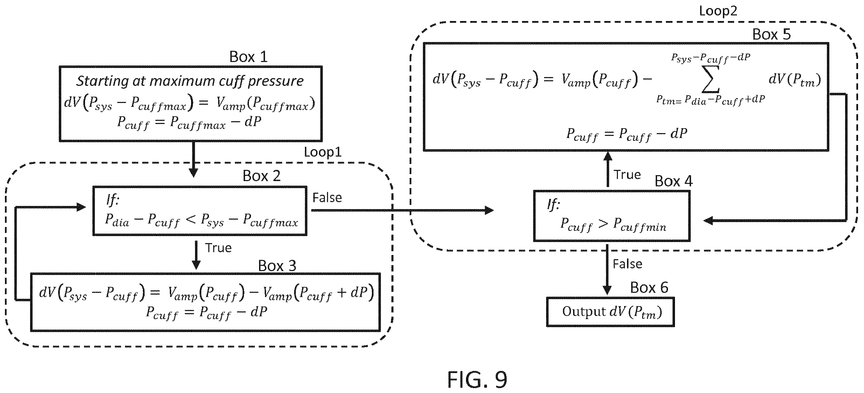

The steps of the algorithm are shown in Fig. 9. In Fig. 9, the set step change in pressure $\Delta P$ for each step change is shown as dP. The $\Delta V$ values are shown with the symbol dV. The algorithm comprises a step-wise processing of the $V_{amp}$ signal in set steps, dP, of applied pressure. Each step represents a move from an initial $P_{cuff}$ value of $P_{cuff\_initial}$ to a next $P_{cuff}$ value along the signal of $P_{cuff}$ initial - dP.

The algorithm starts at the maximum applied pressure and then moves along the $V_{amp}$ signal in steps dP, recording a new dV value in the array for each step. Each dV value is also recorded along with a transmural pressure value to which it corresponds. Each dV value corresponds to a change in arterial volume over the given dP interval. The recorded transmural pressure value for each dV value is the value shown in brackets after 'dV' in Fig. 9. The transmural pressure value for each dP interval is set as equal to $P_{tm} = P_{sys\_initial} - P_{cuff\_initial}$, where $P_{sys\_initial}$ is the systolic blood pressure value for the blood pulse at the location of $P_{cuff\_initial}$. After a given dV value is assigned, the variable "$P_{cuff}$" is incremented to the next $P_{cuff}$ value $P_{cuff} - dP$, ready to assign the next dV value.

There are six boxes, which are each labelled in Fig. 9. There are also two repeat loops which are each indicated by dashed boxes and labelled Loop 1 and Loop 2.

The steps performed by the different boxes will now be explained.

In box 1, the algorithm is initiated by setting a first element of the dV array as equal simply to the $V_{amp}$ signal value at the maximum applied cuff pressure (i.e. starting pressure). The $P_{tm}$ value for this first dV value will be equal to $P_{sys}$ for the pulse occurring at the maximum cuff pressure minus the value of $P_{cuff}$ at the maximum applied cuff pressure, $P_{cuffmax}$.

The algorithm then moves to box 2. As shown in Fig. 9, the algorithm comprises two major loops: loop 1 and loop 2. Each of the loops assigns a portion of the dV values in the array. Loop 1 applies only while $P_{cuff}$ (the applied pressure by the cuff) remains high enough for the artery to be collapsed (i.e. completely occluded) at diastole (when arterial pressure is lowest). When this condition no longer applies, the algorithm moves to loop 2 which assigns the remainder of the dV values.

Box 2 checks whether the artery collapse condition still applies at diastole, by checking whether the transmural pressure at diastole for the particular $P_{cuff}$ value is less than some artery collapse threshold. In the example of Fig. 9, this threshold is conveniently set as equal to the transmural pressure at systole of the first pulse in the $V_{amp}$ signal. As explained above, the cuff pressure is set so that at its starting applied pressure, $P_{cuffmax}$, the artery is completely occluded at both systole and diastole, before pressure is gradually decreased. Hence it is known that the artery collapse condition applies at systole for $P_{cuffmax}$.

If the artery collapse condition is found to apply at diastole, the algorithm moves to Box 3. In Box 3, the next dV value in the array is simply set as equal to the change in the volume amplitude signal between the current applied pressure value $P_{cuff}$ and the immediately preceding applied pressure value $P_{cuff} + dP$. This change in the volume amplitude signal represents a change in absolute arterial volume, $V_{art}$, across the step interval dP. This can be understood as follows.

Loop 1 (and hence Box 3) only applies for the range of the applied cuff pressures for which $V_{art}$ at diastole is zero (i.e. where the artery is completely occluded at diastole). Hence, for the whole span of the $V_{amp}$ signal for which loop 1 applies, $V_{art}$ at diastole is the same (equal to zero) at each and every $P_{cuff}$ position. Further, each $V_{amp}$ value is

equal to pulse volume: the difference between the arterial volume at systole and at diastole. Since $V_{art\_diastole}$ is equal to zero, each $V_{amp}$ value over this initial span is just representative of $V_{art\_systole}$, i.e. absolute arterial volume at systole, at the particular $P_{cuff}$ position concerned. So change in $V_{amp}$ for each dP step is equal to change in absolute arterial volume at systole for so long as the artery remains fully occluded at diastole.

Once the dV value has been assigned by Box 3, Box 3 also then increments the $P_{cuff}$ variable to the next $P_{cuff}$ value after a new interval of dP. $P_{cuff}$ is thus changed to $P_{cuff} = P_{cuff}$-dP. The algorithm then loops back to Box 2.

Once the artery collapse condition no longer applies at diastole (as determined by a false outcome of Box 2), the algorithm moves to Loop 2. Loop 2 assigns dV values of the array for the remainder of the $V_{amp}$ signal, i.e. the remaining span of the $V_{amp}$ signal for which absolute arterial volume at diastole is no longer constant across all applied pressures, but will vary for the different dV values. This means that for this remaining part of the $V_{amp}$ signal, the dV value cannot simply be set as equal to change in the $V_{amp}$ signal occurring over the dP interval, as both $V_{art\_sys}$ and $V_{art\_dia}$ must be taken into account in the calculation. In other words, for this part of the $V_{amp}$ signal, change in $V_{amp}$ (change in arterial pulse volume) for a given dP interval will not correspond to a correct change in arterial volume for the particular dP interval, because both the lower and the upper values of the pulse may have moved compared to the last pulse.

For Loop 2 therefore, a more complex procedure must be performed to determine the dV value. In particular, a sum is performed, as shown in Box 5 which calculates a sum of all of the previously recorded dV values in the array, starting from the value recorded for a $P_{tm}$ corresponding to diastole of the current $P_{cuff}$, and up to the value recorded for $P_{tm}$ corresponding to systole of the current $P_{cuff}$ value, minus dP. This is then subtracted from the $V_{amp}$ value for the current $P_{cuff}$ position, and assigned as the dV value for a $P_{tm}$ corresponding to the systole point of the pulse at the current $P_{cuff}$ position. This results in a dV value equal to change in absolute arterial volume at systole over the current dV interval. This can be understood as follows.

Each iteration of Box 3 or Box 5 results in determining a new dV value which is equal to change in absolute arterial volume at systole over a particular dP interval. The sum in Box 5 effectively considers an artificial arterial volume pulse having the same systolic and diastolic pressure values as the pulse at the current $P_{cuff}$ position, less dP, i.e. a pulse shifted down by dP. The sum then adds together all of the previous absolute arterial volume changes recorded for dP intervals spanning between the lower and upper most values of this imagined pulse. Then Box 4 subtracts this from the $V_{amp}$ signal value for the pulse at the current $P_{cuff}$ position. The result is a value representative of change in absolute arterial volume at systole over the latest applied pressure interval dP, i.e. subtracting the result of the sum means that the remaining portion of $V_{amp}(P_{cuff})$ must correspond to the 'contribution' to arterial volume provided by the new dP interval.

Loop 2 continues until the end of the $V_{amp}$ signal is reached, i.e. until the minimum $P_{cuff}$ value, $P_{cuffmin}$ is reached. Box 4 checks on each iteration whether this point has been reached. If $P_{cuffmin}$ has not yet been reached then the algorithm returns to Box, and a new dV value recorded, and the $P_{cuff}$ variable incremented to the next value, $P_{cuff}$-dP.

Once $P_{cuff}$ reaches $P_{cuffmin}$, the condition tested by Box 4 no longer applies and the algorithm leaves Loop 2 and moves to the final box, Box 6. Box 6 outputs the resulting complete array $dV(P_{tm})$, which includes all of the recorded arterial volume changes and the $P_{tm}$ value to which each corresponds.

Further integration and differentiation steps are subsequently necessary to obtain the final measure of arterial compliance. These will be explained further below.

Fig. 9 represents just one example algorithm for obtaining the dataset of ΔV values.

By way of a further example, and as mentioned previously, the set of ΔV values may alternatively be obtained based on use of the absolute arterial volume signal, $V_{art}$ (Fig. 5). This signal can be processed in step-wise steps ΔP of applied pressure. Due to the relatively slow change in applied pressure compared to faster arterial pressure oscillations, each new ΔP position in the volume signal will coincide with a new particular pulse cycle. The ΔV value corresponding to each step may be taken simply as the change in arterial volume between systole points of the pulses or diastole points of the pulses). $P_{tm}$ values corresponding to each ΔV value can be calculated in a similar way to the algorithm of Fig. 9, each being set as equal to $P_{sys}$ for the particular pulse minus the applied pressure value at the location of that pulse in the $V_{art}$ signal. This results in a dataset of values ΔV corresponding to changes in arterial volume for a set change ΔP in applied pressure at different transmural pressure values.

The output of the processing procedure applied to the first signal is an array $dV(P_{tm})$ which represents the arterial volume changes caused by a set pressure change of value dP at different transmural pressures, $P_{tm}$.

A plot of arterial volume as a function of transmural pressure may be obtained by then numerically integrating the values of the obtained array $dV(P_{tm})$ with respect to transmural pressure.

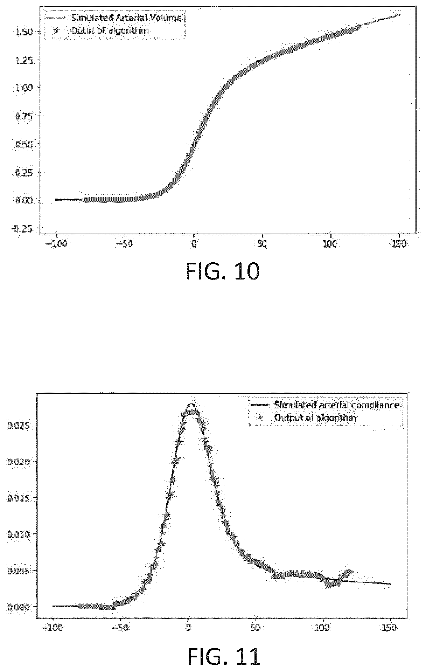

Fig. 10 illustrates an example output of such an integration, and shows the resulting relationship between arterial volume and transmural pressure. The y-axis corresponds to arterial volume. The x-axis corresponds to transmural pressure. Fig. 10 shows the output of the integration as a plotted series of stars. The graph of Fig. 10 also shows, for comparison, the simulated (correct) arterial volume (shown as a solid line). As can be seen from Fig. 10, there is a high degree of agreement between the two.

This obtained function may then be differentiated with respect to transmural pressure to obtain a function of arterial compliance as a function of transmural pressure. An example such a derived function is shown in Fig. 11.

Fig. 11 again shows the simulated (correct) arterial compliance function (shown as a solid line), with the output of the differentiation shown as a series of stars plotted on the graph. Again, it can be seen that there is a high degree of agreement between the two.

The integrations involved in this method may be performed directly by processing the integrands with very small steps. Alternatively, more complex or in-depth methods of integration may be employed, depending upon the degree of accuracy or precision which is required. Such methods may for example be based on a better approximation of the integrand by means of interpolation, or by combining approximations obtained with different step sizes (Romberg method), or by applying methods developed for differential equations (Runge-Kutta). These represent examples only. The skilled person will be various aware of methods for performing numerical integration, any of which may be used.

The above described method analyses arterial volume changes detected during an inflation of the blood pressure cuff. As the cuff pressure reaches systolic values the artery under the cuff collapses. In practice, after the moment of arterial collapse, volume changes are still detected with the cuff setup. This occurs as a result of blood ejected from the heart hitting against the closed portion of the artery. This is known as a knocking effect.

The accuracy of the derived measure of arterial compliance may, according to certain embodiments be further improved by taking this knocking effect into account.

The knocking effect may be quantified according to one or more embodiments.

In one set of examples, the relationship between arterial volume and transmural pressure may be derived independently by a further alternative method to that outlined above. Thus two arterial volume vs transmural pressure functions may be derived, using different methods. Any difference between the derived relationships may be attributed to the knocking effect. Hence, a quantification of the knocking effect may be derived based on any derived difference between the two derived arterial volume vs transmural pressure relationships.

By way of one set of examples, arterial volume as a function of transmural pressure can be derived based on models which describe mechanical principles of arterial collapse.

An example of such an arterial mechanics model is described for example in the paper: Drzewiecki G et al (1994). Theory of the oscillometric maximum and the systolic and diastolic detection ratios. Annals of Biomedical Engineering, 22, pp 88-96.

The parameters of such a model may for example can be fitted to the output of the procedure described above (e.g. comprising the algorithm of Fig. 9 or a different algorithm). The differences between the output of the parametric model and the non-parametric computation of arterial volume at negative transmural pressures is indicative of the knocking effect.

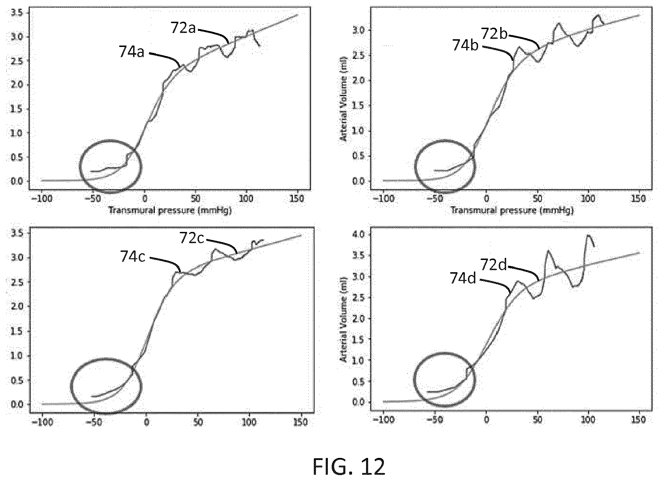

Fig. 12 shows a set of example arterial volume (y-axis) vs transmural pressure (x-axis) relationships derived using both the example algorithm outlined above (Fig. 9) and using a parametric model based method. Each graph of Fig. 12 shows the results from both methods. The relationships derived using a parametric model based method are labelled 72a-72d respectively. The relationships derived using the algorithm labelled 74a-74d respectively. The differences in the outputs between the non-parametric based method and the parametric model based method in each graph can be seen. The circles in each graph indicate regions which are affected by the knocking effect.

This information may be used when interpreting data recorded by means of oscillometric measurements for the purpose of more accurately measuring blood pressure or for obtaining true arterial volume under the cuff.

In a simple example for instance, if a knocking effect is found to be unusually large (e.g. exceeding a pre-defined threshold level) then the oscillometric based measurement may be interpreted as inaccurate, and a further alternative blood pressure measurement approach followed or suggested to the clinician. For example, an invasive blood pressure measurement means may be used in the event that the knocking effect is found to exceed the pre-defined threshold.

In other examples for instance, a quantification of the knocking may be derived, and this might be used to post-process the oscillometric-based measurement, for instance to correct or compensate for the effect. In further examples, a model might be applied capable of taking as an input the oscillometric and parametric-derived data, and the quantified knocking effect and outputting an improved signal with the knocking effect reduced or eliminated. A machine learning model may be used for example.

Although in examples outlined above, the oscillometric blood pressure measurement device is a sphygmomanometer type device comprising an inflatable cuff, this represents just one advantageous example. Any alternative form of oscillometric blood pressure measurement device may alternatively by used. The measurement device is preferably capable of applying a varying applied pressure to an artery of a subject in use. This may be through any pressure application means.

Examples in accordance with a further aspect of the invention provide a control unit in accordance with any example or embodiment outlined above or described below, or in accordance with any claim of this application, and an oscillometric blood pressure measurement device operatively coupled to the control unit.

In some embodiments, the system may further comprise a patient monitor unit, wherein the patient monitor unit comprises the control unit.

In some embodiments, the system may be a patient monitoring system. It may for example comprise one or more input ports for receiving data from one or more further auxiliary units, e.g. medical sensing devices.

Examples in accordance with a further aspect of the invention provide a method of deriving a measure of arterial compliance, comprising:

obtaining based on use of an oscillometric blood pressure measurement device a first signal indicative of a variation in arterial volume or arterial pulse volume of an artery assumed to be in contact with the measurement device, as a pressure applied to the artery by the measurement device is varied across a range of pressures;

further obtaining using the oscillometric blood pressure measurement device one or more systolic and diastolic blood pressure measurement values;

based on the first signal, and the acquired blood pressure measurement values, compiling a dataset of values, ΔV, representative of the change in arterial volume for set step changes, ΔP, in applied pressure, at different transmural pressure values; and

numerically integrating the dataset of ΔV values to derive a function of arterial volume with transmural pressure, and differentiating said function of arterial volume with respect to transmural pressure, to thereby derive a function of arterial compliance with transmural pressure.

Implementation options and details for each of the above steps may be understood and interpreted in accordance with the explanations and descriptions provided above for the apparatus aspect of the present invention (i.e. the control unit aspect).

Any of the examples, options or embodiment features or details described above in respect of the apparatus aspect of this invention (in respect of the control unit) may be applied or combined or incorporated into the present method aspect of the invention.

As discussed above, embodiments make use of a control unit. The control unit may be a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A control unit (12) for deriving a measure of arterial compliance, operably coupleable, when in use, with an oscillometric blood pressure measurement device (14),
the control unit (12) adapted to:

acquire based on use of the oscillometric blood pressure measurement device a first signal indicative of a variation in arterial volume or arterial pulse volume of an artery assumed to be in contact with the measurement device, as a pressure applied to the artery by the measurement device is varied across a range of pressures; further acquire using the oscillometric blood pressure measurement device one or more systolic and diastolic blood pressure measurement values;

based on the first signal and acquired blood pressure measurement values, compile a dataset of values, $\Delta V$, representative of the changes in arterial volume for set step changes, $\Delta P$, in applied pressure, at different transmural pressure values, and

numerically integrate the dataset of $\Delta V$ values to derive a function of arterial volume with transmural pressure, and differentiate said function of arterial volume with respect to transmural pressure, to thereby derive a function of arterial compliance with transmural pressure.

2. The control unit according to claim 1, wherein compiling the dataset of $\Delta V$ values comprises a stepwise processing of the first signal, stepping through the first signal in successive step intervals, $\Delta P$, of applied pressure.

3. The control unit according to claim 2, wherein the compiling the dataset of $\Delta V$ values further comprises determining a transmural pressure value corresponding to each of the successive step intervals.

4. The control unit according to any of claims 1-3, wherein transmural pressure values to which each of the $\Delta V$ values correspond are determined based on the measured systolic and/or diastolic blood pressure values and based on the applied pressure value to which the respective $\Delta V$ value corresponds.

5. The control unit according to any of claims 1-4, wherein the $\Delta P$ interval is selected such that the $\Delta V$ values correspond to changes in arterial volume between different heart pulses represented in the first signal.

6. The control unit according to any of claims 1-5, wherein the first signal is a peak-to-peak volume amplitude signal, $V_{amp}$, indicative of a peak-to-peak amplitude of arterial volume oscillations over said range of applied pressures.

7. The control unit according to claim 6, wherein the set of values $\Delta V$ are based on changes in the volume amplitude function, $V_{amp}$, for said set step changes, $\Delta P$, in applied pressure, at different transmural pressure values.

8. The control unit according to any of claims 1-7, wherein the oscillometric blood pressure measurement device comprises a fluid-inflatable cuff.

9. The control unit according to claim 8, wherein, in use, the control unit is configured in use to implement said variation in applied pressures across a range of pressures by controlling the inflatable cuff to gradually inflate or deflate through a range of internal fluid pressures of the inflatable cuff.

10. The control unit according to claim 8 or 9, wherein said applied pressure to the artery is taken to be equal to a baseline value of an internal fluid pressure of the inflatable cuff.

11. The control unit according to any of claims 8-10, wherein the first signal is obtained based on a combination of: measured oscillations in an internal fluid pressure of the fluid-inflatable cuff at each applied pressure value, and a pre-determined internal fluid pressure vs internal fluid volume relationship.

12. The control unit according to any of claims 8-11, wherein the pre-determined internal fluid pressure vs internal fluid volume relationship utilizes a pre-determined compliance or elasticity of the fluid-inflatable cuff.

13. A system (10) for deriving a measure of arterial compliance, comprising
a control unit (12) as claimed in any of claims 1-12; and
an oscillometric blood pressure measurement device operatively coupled to the control unit.

14. The system according to claim 13, further comprising a patient monitor unit, wherein the patient monitor unit comprises the control unit.

15. A method of deriving a measure of arterial compliance, comprising:

obtaining based on use of an oscillometric blood pressure measurement device a first signal indicative of a variation in arterial volume or arterial pulse volume of an artery assumed to be in contact with the measurement device, as a pressure applied to the artery by the measurement device is varied across a range of pressures; further obtaining using the oscillometric blood pressure measurement device one or more systolic and diastolic blood pressure measurement values;

based on the first signal, and the acquired blood pressure measurement values, compiling a dataset of values, $\Delta V$, representative of the change in arterial volume for set step changes, $\Delta P$, in applied pressure, at different transmural pressure values; and

numerically integrating the dataset of $\Delta V$ values to derive a function of arterial volume with transmural pressure, and differentiating said function of arterial volume with respect to transmural pressure, to thereby derive a function of arterial compliance with transmural pressure.

FIG. 1

FIG. 2

FIG. 3

FIG. 4

FIG. 5

FIG. 6

FIG. 7

FIG. 8

Box 1

$$Starting\ at\ maximum\ cuff\ pressure$$
$$dV\big(P_{sys} - P_{cuffmax}\big) = V_{amp}(P_{cuffmax})$$
$$P_{cuff} = P_{cuffmax} - dP$$

Loop1

Box 2

*If:*
$$P_{dia} - P_{cuff} < P_{sys} - P_{cuffmax}$$

False

True

Box 3

$$dV\big(P_{sys} - P_{cuff}\big) = V_{amp}\big(P_{cuff}\big) - V_{amp}\big(P_{cuff} + dP\big)$$
$$P_{cuff} = P_{cuff} - dP$$

Loop2

Box 5

$$dV\big(P_{sys} - P_{cuff}\big) = V_{amp}\big(P_{cuff}\big) - \sum_{P_{tm}= P_{dia}-P_{cuff}+dP}^{P_{sys}-P_{cuff}-dP} dV(P_{tm})$$
$$P_{cuff} = P_{cuff} - dP$$

True

Box 4

*If:*
$$P_{cuff} > P_{cuffmin}$$

False

Box 6

$$Output\ dV(P_{tm})$$

FIG. 9

FIG. 10

FIG. 11

FIG. 12

Europäisches
Patentamt

European
Patent Office

Office européen
des brevets

# EUROPEAN SEARCH REPORT

**Application Number**

EP 19 18 4049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HIDEHIKO KOMINE ET AL: "Non-invasive assessment of arterial stiffness using oscillometric blood pressure measurement", BIOMEDICAL ENGINEERING ONLINE, BIOMED CENTRAL LTD, LONDON, GB, vol. 11, no. 1, 10 February 2012 (2012-02-10), page 6, XP021125197, ISSN: 1475-925X, DOI: 10.1186/1475-925X-11-6 * * Title * * abstract * * Curve relationship between cuff pressure and arterial volume; page 2 - page 4; figures 1, 2 * * Carotid arterial compliance; page 5 - page 6 * * page 7; figure 3 * * page 9, paragraph 2 * ----- | 1-15 | INV. A61B5/02 A61B5/022 |
| | | | TECHNICAL FIELDS SEARCHED (IPC) |
| | | | A61B |

The present search report has been drawn up for all claims

| Place of search | Date of completion of the search | Examiner |
|---|---|---|
| The Hague | 7 January 2020 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DRZEWIECKI G et al.** Theory of the oscillometric maximum and the systolic and diastolic detection ratios. *Annals of Biomedical Engineering,* 1994, vol. 22, 88-96